# FASCICULE DE BREVET EUROPEEN

(11) **EP 2 525 894 B1**
(45) Date de publication et mention de la délivrance du brevet: **21.06.2017**
(21) Numéro de dépôt: 11700189.1
(22) Date de dépôt: 14.01.2011
(51) Int. Cl.: C07C 29/50, C07C 35/08, C07C 45/33, C07C 49/403, C07C 51/31, C07C 55/14, B01D 53/14

(54) **PROCEDE D'OXYDATION D'HYDROCARBURES**
VERFAHREN ZUR OXIDIERUNG VON KOHLENWASSERSTOFFEN
METHOD FOR OXIDIZING HYDROCARBONS

(30) Priorité: 21.01.2010 FR 1050386
(43) Date de publication de la demande: 28.11.2012
(73) Titulaire: Rhodia Operations, 93306 Aubervilliers (FR)
(72) Inventeur: GALINAT, Sophie, F-69008 Lyon (FR); VERACINI, Serge, F-69005 Lyon (FR); IGERSHEIM, Françoise, F-69003 Lyon (FR)
(74) Mandataire: Chatelan, Florence Anne
(86) Numéro de dépôt international: PCT/EP2011/050469
(87) Numéro de publication internationale: WO 2011/089074

(56) Documents cités:
- FR-A- 1 230 366
- US-A- 4 102 983
- US-A- 5 505 920
- US-A- 5 772 734
- DATABASE WPI Week 200338 Thomson Scientific, London, GB; AN 2003-397676 XP002597323, & JP 2003 047823 A (TEIJIN LTD) 18 février 2003 (2003-02-18)
- DATABASE WPI Week 200820 Thomson Scientific, London, GB; AN 2008-C73736 XP002597324, & RU 2 316 385 C1 (TSEGELSKII V G) 10 février 2008 (2008-02-10)

## Description

La présente invention concerne un procédé d'oxydation d'hydrocarbures, notamment d'hydrocarbures saturés pour la production de peroxydes, alcools, cétones, aldéhydes et/ou diacides.

Elle se rapporte plus particulièrement à un procédé d'oxydation par l'oxygène moléculaire d'un hydrocarbure saturé cycloaliphatique pour la production de cétones/alcools et encore plus particulièrement à l'oxydation du cyclohexane par l'oxygène moléculaire en cyclohexanol et cyclohexanone.

Le mélange de cyclohexanol et cyclohexanone ou l'un de ces produits sont utilisés pour la synthèse d'acide adipique ou d'epsilon caprolactame.

Le procédé de fabrication de cyclohexanol, cyclohexanone par oxydation du cyclohexane par l'oxygène moléculaire ou un gaz contenant de l'oxygène moléculaire en présence ou non de catalyseur est décrit dans de nombreux brevets et nombreuses publications tels que, par exemple, les brevets GB 777087, 1112837, 964869, 1191573, US 3479394, US 4877903.

Généralement, dans un tel procédé d'oxydation par l'oxygène moléculaire, le taux de transformation de l'hydrocarbure saturé, tel que le cyclohexane, est volontairement maintenu à une valeur faible pour améliorer la sélectivité de la réaction en produits oxydés valorisables, notamment transformables en cyclohexanol et cyclohexanone.

Par ailleurs, cette sélectivité est meilleure et ne peut être maintenue à une valeur acceptable que si la concentration en produits oxydés dans le milieu réactionnel est maintenue à une valeur basse.

En conséquence, la réaction d'oxydation est réalisée en utilisant un milieu réactionnel présentant une concentration en hydrocarbure élevée, cet hydrocarbure jouant le rôle de solvant. Pour l'économie du procédé, il est nécessaire de récupérer cet hydrocarbure non oxydé en fin de réaction, pour le recycler dans l'étape d'oxydation et donc constituer une boucle de circulation de l'hydrocarbure.

En outre, le procédé d'oxydation de l'hydrocarbure en peroxyde et/ou alcool ou cétone comprend des étapes de réaction et de séparation des produits contenus dans le milieu réactionnel avec élimination des fractions dites de « produits légers », c'est-à-dire les produits ayant un point d'ébullition inférieur à celui de l'hydrocarbure.

Ces différentes fractions de produits à bas point d'ébullition sont souvent appelées dans de tels procédés des « off-gaz » et sont destinées à être détruites par incinération ou brûlées dans une torchère.

Toutefois, bien que l'efficacité des étapes de séparation soit élevée, les « off-gaz » récupérés contiennent toujours une faible quantité d'hydrocarbure non oxydé qu'il est très avantageux de récupérer tant d'un point de vue de sauvegarde de l'environnement que de l'économie du procédé.

Il existe donc un besoin d'un procédé permettant de récupérer de manière économique et sélective l'hydrocarbure saturé présent dans ces « off-gaz » pour pouvoir le recycler dans le procédé d'oxydation.

On connaît de par le document US 5,505,920, un procédé d'oxydation du cyclohexane en acide adipique, dans lequel les effluents gazeux sont traités avec de l'acide acétique afin de séparer le cyclohexane résiduel. Ce procédé est particulièrement adapté au traitement de gaz résiduaires générés pendant l'oxydation de cyclohexane en acide adipique utilisant l'acide acétique comme solvant qui est retourné en même temps que le cyclohexane récupéré, au réacteur d'oxydation du cyclohexane en acide adipique.

Le document US 4,102,983 décrit une méthode pour éliminer des matériaux organiques volatiles d'un mélange gazeux d'air et de vapeurs de matériaux organiques volatiles déchargés à l'atmosphère lors du stockage, du chargement ou du transport de tels matériaux, purifiant par conséquent le mélange de gaz déchargés à l'atmosphère. Ce procédé utilise un mélange liquide ayant une excellente capacité d'absorber et de séparer des matériaux organiques volatiles. Ce mélange comporte une huile minérale raffinée et des composés organiques particuliers tels que notamment les esters phtaliques, siliciques, phosphoriques ou carboxyliques

Il existe donc toujours un besoin de proposer un procédé permettant de traiter les off-gaz d'un procédé d'oxydation d'hydrocarbures saturés tels que l'oxydation de cyclohexane en cyclohexanol/cyclohexanone pour récupérer l'hydrocarbure saturé présent dans ces off-gaz et le recycler dans le procédé d'oxydation.

A cet effet, l'invention propose un procédé d'oxydation d'hydrocarbure saturé par l'oxygène moléculaire comprenant un procédé de traitement des effluents gazeux produits par ledit procédé d'oxydation, ledit procédé de traitement comprend une étape de mise en contact des effluents gazeux à traiter avec une huile à l'état liquide pour absorber l'hydrocarbure saturé contenu dans les effluents et une seconde étape de traitement de l'huile chargée en hydrocarbure par stripping (entraînement) à la vapeur d'eau pour extraire l'hydrocarbure, condensation de la vapeur récupérée et séparation de l'hydrocarbure par décantation.

Le procédé de l'invention est un procédé d'oxydation d'un hydrocarbure saturé comprenant un procédé de traitement des off-gaz produits par le procédé d'oxydation et les étapes de séparation des différents produits, consistant à récupérer l'hydrocarbure présent dans ces off-gaz par absorption dans une huile choisie dans le groupe comprenant les huiles naphténiques et les mélanges d'huiles naphténiques et d'huiles paraffiniques, puis à traiter cette huile contenant l'hydrocarbure par un « stripping » (entraînement) à la vapeur d'eau pour extraire et récupérer l'hydrocarbure absorbé et après séparation, par exemple par condensation et décantation, recycler ledit hydrocarbure dans l'étape d'oxydation.

Selon une caractéristique de l'invention, l'hydrocarbure est choisi dans le groupe comprenant le cyclohexane, le cyclooctane, le cyclododécane, et la décaline.

Le procédé d'oxydation peut être un procédé d'oxydation d'un hydrocarbure en hydroperoxyde d'alkyle, en présence ou non de catalyseur, puis transformation de cet hydroperoxyde d'alkyle en cétone et/ou alcool. Le procédé d'oxydation de l'invention peut également être un procédé d'oxydation, en présence d'un catalyseur, par l'oxygène de l'hydrocarbure en alcool et cétone en une seule étape. Le procédé de l'invention s'applique également aux traitements des off-gaz récupérés dans un procédé d'oxydation par l'oxygène moléculaire d'un hydrocarbure saturé en diacide, comme l'oxydation directe du cyclohexane en acide adipique.

Dans ces procédés, l'oxydation est réalisée avec une quantité importante d'hydrocarbure qui joue le rôle de réactif et de solvant pour éviter que la concentration en produits oxydés soit très importante dans le milieu réactionnel.

Ces procédés comprennent plusieurs étapes de réaction et de séparation des produits, notamment de distillation du cyclohexane en excès pour le recycler à l'étape d'oxydation.

Au cours de ces étapes, il est fréquent de récupérer une fraction gazeuse comprenant notamment les produits de faible point d'ébullition par rapport au point d'ébullition de l'hydrocarbure saturé et non valorisables. Ces fractions gazeuses forment l'essentiel des effluents gazeux de ces procédés d'oxydation et sont souvent désignés par l'expression « off-gaz ». Ces off-gaz sont généralement brûlés, par exemple par une torchère.

Selon le procédé de l'invention, les effluents gazeux ou off-gaz sont traités pour extraire et récupérer la faible quantité d'hydrocarbure saturé présent et ainsi pouvoir la recycler dans le procédé. De plus, cette récupération de l'hydrocarbure diminue les quantités de rejet et donc est favorable à la sauvegarde de l'environnement.

Selon l'invention, ce traitement consiste à faire passer les off-gaz ou effluents dans une huile à l'état liquide, par exemple dans une colonne de lavage ou d'échange gaz/liquide.

Quand l'huile est saturée d'hydrocarbure ou atteint un niveau défini de concentration, l'hydrocarbure absorbé est récupéré par extraction avec de la vapeur d'eau. Ainsi, l'huile est « strippée » (entraînée) par de la vapeur d'eau, le mélange hydrocarbure/vapeur d'eau est condensé. L'hydrocarbure est récupéré par décantation.

L'huile convenable pour le procédé de l'invention doit être choisie pour certaines de ces propriétés listées ci-dessous :
- avoir un point d'ébullition plus élevé que celui de l'hydrocarbure saturé,
- être difficilement oxydable par l'oxygène pour éviter et limiter les phénomènes de dégradation de l'huile,
- être un solvant pour l'hydrocarbure saturé,
- présenter une faible tension de vapeur à la température de « stripping » par la vapeur d'eau,
- présenter une propriété de démixtion, par décantation en présence d'eau, suffisante pour permettre une séparation eau/huile compatible avec une exploitation industrielle.

Parmi les différentes huiles disponibles, les huiles naphténiques et les mélanges d'huiles naphténiques et d'huiles paraffiniques sont particulièrement convenables pour la réalisation du procédé de l'invention.

Le procédé de l'invention permet ainsi de récupérer une quantité non négligeable d'hydrocarbure qui peut être évaluée de quelques dixièmes de pour cent à quelques pour cent de l'hydrocarbure engagé dans le procédé d'oxydation.

Le lavage des effluents gazeux par l'huile et le « stripping » à la vapeur d'eau de l'huile peuvent être réalisés dans tout dispositif adapté connu de l'homme du métier tel que des colonnes d'échange gaz/liquide, des colonnes à distiller à remplissage ou à plateaux, par exemple.

D'autres détails et avantages de l'invention apparaitront plus clairement dans les exemples donnés ci-dessous à titre indicatif.

### Exemple 1 :

Dans un procédé d'oxydation du cyclohexane en cyclohexanol et cyclohexanone, les gaz à rejeter ou off-gaz provenant des réacteurs et des différentes colonnes de séparation sont rassemblés. La concentration en cyclohexane dans ces gaz est comprise entre 8 et 12% en volume.

Ces gaz sont traités avec une huile paraffinique telle que l'huile commercialisée par la société BP France sous la dénomination commerciale Enerthene® 2367, alimentée à une température de 25°C dans une colonne de lavage comprenant des plateaux à cloches. Les gaz sortant de la colonne de lavage ne contiennent plus que 0,3% en volume de cyclohexane.

L'huile chargée en cyclohexane est alimentée, après préchauffage à une température de 110°C, dans la partie haute d'une une colonne de distillation à plateaux à clapets fonctionnant à une pression de 1,2 bars.

De la vapeur d'eau est alimentée en pied de colonne.

L'azéotrope entre l'eau et le cyclohexane est récupéré en tête de colonne et condensé.

Les condensats sont introduits dans un décanteur. La phase organique récupérée est composée de cyclohexane et d'impuretés légères. Cette phase organique est distillée pour éliminer les impuretés légères. Le cyclohexane récupéré est recyclé dans le procédé d'oxydation.

### Exemple 2 :

Dans un procédé d'oxydation du cyclohexane en cyclohexanol et cyclohexanone, les gaz à rejeter ou off-gaz provenant des réacteurs et des différentes colonnes de séparation sont rassemblés. La concentration en cyclohexane dans ces gaz est comprise entre 5 et 7% en poids.

Ces gaz sont traités avec une huile paraffinique et naphténique telle que l'huile commercialisée par la société SHELL sous la dénomination commerciale SHELL Edelex 912, alimentée à une température de 20°C dans une colonne de lavage comprenant des plateaux à cloches. Les gaz sortant de la colonne de lavage ne contiennent plus que 0,12 % en poids de cyclohexane.

L'huile chargée en cyclohexane est alimentée, après préchauffage à une température de 110°C, dans la partie haute d'une une colonne de distillation à plateaux à clapets fonctionnant à une pression de 1,2 bars.

De la vapeur d'eau est alimentée en pied de colonne.

L'azéotrope entre l'eau et le cyclohexane est récupéré en tête de colonne et condensé.

Les condensats sont introduits dans un décanteur. La phase organique récupérée est composée de cyclohexane et d'impuretés légères. Cette phase organique est distillée pour éliminer les impuretés légères. Le cyclohexane récupéré est recyclé dans le procédé d'oxydation.

### Exemple 3 :

Dans un procédé d'oxydation du cyclohexane en cyclohexanol et cyclohexanone, les gaz à rejeter ou off-gaz provenant des réacteurs et des différentes colonnes de séparation sont rassemblés. La concentration en cyclohexane dans ces gaz est comprise entre 7 et 9 % en poids.

Ces gaz sont traités avec une huile paraffinique et naphténique telle que l'huile commercialisée par la société SHELL sous la dénomination commerciale SHELL Edelex 912, alimentée à une température de 22°C dans une colonne de lavage comprenant des plateaux à cloches. Les gaz sortant de la colonne de lavage ne contiennent plus que 0,22 % en poids de cyclohexane.

L'huile chargée en cyclohexane est alimentée, après préchauffage à une température de 110°C, dans la partie haute d'une une colonne de distillation à plateaux à clapets fonctionnant à une pression de 1,2 bars.

De la vapeur d'eau est alimentée en pied de colonne.

L'azéotrope entre l'eau et le cyclohexane est récupéré en tête de colonne et condensé.

Les condensats sont introduits dans un décanteur. La phase organique récupérée est composée de cyclohexane et d'impuretés légères. Cette phase organique est distillée pour éliminer les impuretés légères. Le cyclohexane récupéré est recyclé dans le procédé d'oxydation.

## Revendications

1. Procédé d'oxydation d'hydrocarbure saturé par l'oxygène moléculaire comprenant un procédé de traitement des effluents gazeux produits par ledit procédé d'oxydation, **caractérisé en ce que** :
- ledit procédé de traitement comprend une étape de mise en contact des effluents gazeux à traiter avec une huile à l'état liquide pour absorber l'hydrocarbure saturé contenu dans les effluents et une seconde étape de traitement de l'huile chargée en hydrocarbure par stripping (entraînement) à la vapeur d'eau pour extraire l'hydrocarbure, condensation de la vapeur récupérée et séparation de l'hydrocarbure par décantation,
- l'hydrocarbure est choisi dans le groupe comprenant le cyclohexane, le cyclooctane, le cyclododécane et la décaline, et
- l'huile est choisie dans le groupe comprenant les huiles naphténiques et les mélanges d'huiles naphténiques et d'huiles paraffiniques.

2. Procédé selon la revendication 1, **caractérisé en ce qu'**il est un procédé d'oxydation d'un hydrocarbure saturé en hydroperoxyde, alcool et/ou cétone.

3. Procédé selon l'une des revendications 1, **caractérisé en ce qu'**il est un procédé d'oxydation par l'oxygène du cyclohexane en acide adipique.

## Patentansprüche

1. Verfahren zur Oxidation eines gesättigten Kohlenwasserstoffrests mit molekularem Sauerstoff, umfassend ein Verfahren zur Behandlung von bei dem Oxidationsverfahren anfallenden gasförmigen Austragsströmen, **dadurch gekennzeichnet, dass**:
- das Behandlungsverfahren einen Schritt des Inberührungbringens der zu behandelnden gasförmigen Austragsströme mit einem Öl in flüssigem Zustand zur Absorption des in den Austragsströmen enthaltenen gesättigten Kohlenwasserstoffs und einen zweiten Schritt der Behandlung des kohlenwasserstoffbeladenen Öls durch Strippen (Schleppen) mit Wasserdampf zur Extraktion des Kohlenwasserstoffs, der Kondensation des gewonnenen Dampfs und der Abtrennung des Kohlenwasserstoffs durch Absetzen umfasst,
- der Kohlenwasserstoff aus der Gruppe umfassend Cyclohexan, Cyclooctan, Cyclododecan und Decalin ausgewählt wird und
- das Öl aus der Gruppe umfassend naphtenische Öle und Mischungen von naphthenischen Ölen und paraffinischen Ölen ausgewählt wird.

2. Verfahren nach Anspruch 1, **dadurch gekennzeichnet, dass** es sich um ein Verfahren zur Oxidation eines gesättigten Kohlenwasserstoffs zum Hydroperoxid, Alkohol und/oder Keton handelt.

3. Verfahren nach einem der Ansprüche 1, **dadurch gekennzeichnet, dass** es sich um ein Verfahren zur Oxidation von Cyclohexan mit Sauerstoff zu Adipinsäure handelt.

## Claims

1. Process for the oxidation of a saturated hydrocarbon via molecular oxygen comprising a process for treating gaseous effluents produced by said oxidation process, **characterized in that**:
- said treatment process comprises a step of bringing the gaseous effluents to be treated into contact with an oil in the liquid state in order to absorb the saturated hydrocarbon contained in the effluents and a second step of treating the hydrocarbon-loaded oil by steam stripping (distillation) in order to extract the hydrocarbon, condensing the vapour recovered and separating the hydrocarbon by settling,
- the hydrocarbon is selected from the group consisting of cyclohexane, cyclooctane, cyclododecane and decalin, and
- the oil is selected from the group consisting of naphthenic oils and mixtures of haphthenic oils and paraffinic oils.

2. Process according to Claim 1, **characterized in that** it is a process for the oxidation of a saturated hydrocarbon to hydroperoxide, alcohol and/or ketone.

3. Process according to one of Claims 1, **characterized in that** it is a process for the oxidation, via oxygen, of cyclohexane to adipic acid.
